# EUROPÄISCHE PATENTANMELDUNG

(11) **EP 1 900 805 A1**
(43) Veröffentlichungstag der Anmeldung: **19.03.2008**
(21) Anmeldenummer: 07017652.4
(22) Anmeldetag: 10.09.2007
(51) Int. Cl.: C12M 1/107

(54) **Fermentationsvorrichtung**

(30) Priorität: 12.09.2006 DE 102006043534
(71) Anmelder: Landesbetrieb Hessisches Landeslabor, 36251 Bad Hersfeld (DE)
(72) Erfinder: Zerr, Walter, 36251 Ludwigsau (DE); Corell, Jean, 36251 Bad Hersfeld (DE)
(74) Vertreter: Buchhold, Jürgen

(57) **Zusammenfassung**

Die Erfindung bezieht sich auf eine Fermentationsvorrichtung 1. Diese umfasst einen Behälter 10, in den Fermentationsmasse und Biomasse über mindestens eine Öffnung einbringbar ist. Sie weist ferner eine Rühreinrichtung zum Rühren von Fermentationsmasse und Biomasse sowie einen Deckel 2 auf. In diesen Deckel 2 ist ausschließlich die Rühreinrichtung und zwar in dessen Mitte eingebracht. Ferner sind der Behälter 10 und der Deckel 2 aus einem Kunststoff geformt.

## Beschreibung

Die Erfindung betrifft eine Fermentationsvorrichtung gemäß dem Oberbegriff von Anspruch 1 sowie ein Verfahren zur Bestimmung von Parametern für die Fermentierung von Biomasse gemäß dem Oberbegriff von Anspruch 18.

In einer erfindungsgemäßen Fermentationsvorrichtung werden Pflanzenmaterialien auf ihren Energiegehalt und auf ihr Gärungsverhalten hin untersucht. Ferner dient sie als Modell- oder Pilotanlage zur Bestimmung von verschiedenen Verfahrensparametern wie Faulraumbelastung, Verweilzeit von Fermentationsmasse und oder Biomasse, usw., die alsdann auf eine größere Biogasanlage übertragbar sind.

Die Biogasherstellung läuft über vier Schritte ab. Zunächst wird das Zellmaterial der Pflanzen durch Bakterien aufgeschlossen und die einzelnen biologischen Makromoleküle, wie Stärke, Proteine, Fette und DNS in einfachere Moleküle wie Zucker, Aminosäuren, Nukleotide und Fettsäuren umgewandelt Diese Substanzen werden in einem zweiten Schritt zu verschiedenen Säuren und Alkoholen abgebaut. Hieraus entsteht in einem dritten Schritt Essigsäure, die in einem vierten Schritt zu Methangas und Kohlendioxid umgesetzt wird.

Für jeden dieser vier Schritte sind unterschiedliche Arten von Bakterien verantwortlich. Wenn es im Fermentationsgefäß zu einer Temperatur- oder Drucksteigerung kommt, können Bakterien, die Essigsäure in Methangas und Kohlendioxid umwandeln, nicht mehr ordnungsgemäß funktionieren und der Fermentationsprozess wird verlangsamt. Das Gleiche gilt, wenn der Fermentationsreaktor mit zuviel Biomasse beladen wird (man spricht dann von zu hoher Faulraumbelastung). In einem solchen Fall laufen die ersten drei Abbauschritte kontinuierlich ab und es entstehen große Mengen an Essigsäure. Weil die Umsetzung von Essigsäure in Methangas und Kohlendioxid sehr langsam abläuft und somit der geschwindigkeitsbestimmende Schritt der Fermentation ist, bilden sich bei zu großen Mengen an Biomasse mit der Zeit ansteigende Mengen an Essigsäure. Hohe Essigsäuregehalte machen jedoch das Reaktionsmedium sauer und bewirken ein teilweises Absterben der in der Fermentationsmasse vorhandenen Bakterien. Insbesondere jene Bakterien, die Essigsäure zu Methan umsetzen, können in stark sauren Medien nicht mehr einwandfrei arbeiten.

Deshalb ist es erforderlich, zum einen Temperatur und Druck im Fermentationsbehälter konstant zu halten und zum anderen keine zu großen Mengen an Biomasse zur Reaktion zu bringen. Ferner ist es von Bedeutung, die Biomasse in einer bestimmten Homogenität bereitzuhalten. Häckselt man diese außerordentlich klein, laufen die einzelnen biochemischen Reaktionen deutlich schneller ab als bei lediglich grob gemahlenem oder gehäckseltem biologischem Schnittgut. Diese Zusammenhänge lassen sich mit einer Fermentationsvorrichtung nach der Erfindung simulieren und quantitativ erfassen.

Der Stand der Technik offenbart bereits nachfolgende Fermentationsvorrichtungen und Verfahren:

Die DE 200 06 467 U1 beschreibt eine Vorrichtung zum Fermentieren von Biomasse mit einem Kessel, in den die Biomasse einbringbar ist, wobei der Kessel von einer Grundfläche und einer die Grundfläche umschließenden Wand gebildet und von einem Deckel abgedeckt ist, der fest mit einem der Grundfläche fernen Bereich der Wand verbunden ist, mit einer Rühreinrichtung zum Rühren der Biomasse_sowie mindestens einer Öffnung zum Einfüllen bzw. Entnehmen von Biomasse. Die Vorrichtung zeichnet sich dadurch aus, dass der Deckel mindestens teilweise von einem elastischen Material gebildet ist. Diese Vorrichtung ist relativ komplex und ermöglicht nur Untersuchungen bei einem bestimmten Überdruck, sofern nicht permanent Biogas entfernt wird. Somit wäre diese Vorrichtung für Reihen-Untersuchungen nicht geeignet. Ferner wurde die Rühreinrichtung außermittig angebracht und kann somit keine homogene Durchmischung garantieren.

Die DE 100 17 436 C2 betrifft eine Vorrichtung zum Fermentieren von Biomasse mit einem Kessel, in dem die Biomasse einbringbar ist, wobei der Kessel von einer Grundfläche und einer die Grundfläche umschließenden Wand gebildet und von einem

Deckel abgedeckt ist, der fest mit einem der Grundfläche fernen Bereich der Wand verbunden ist, mit einer Rühreinrichtung zum Rühren der Biomasse sowie mindestens einer Öffnung zum Einfüllen bzw. Entnehmen von Biomasse. Der Deckel ist von mindestens einer festen, im wesentlichen geschlossenen Materialschicht gebildet, die einen vorherbestimmten Bereich enthält, der von einer Fixiereinrichtung für eine elastische Hülle umschlossen ist, wobei der vorherbestimmte Bereich mindestens eine Bohrung enthält, die eine Kommunikationsverbindung zwischen dem Inneren des Kessels und dem Äußeren des Kessels herstellt, wobei der vorbestimmte Bereich kleiner als der gesamte Deckel ist und sich die Öffnung außerhalb dieses Bereiches im Deckel befindet. Nachteilig an dieser Vorrichtung ist, dass keine Temperatursteuerungsvorrichtung vorgesehen ist. Ferner befindet sich ein vorgesehenes Rührwerk am Rand der Vorrichtung, so dass eine Durchmischung des Fermentiergutes nicht gleichmäßig möglich ist.

Die DE 34 11 264 A1 beschreibt eine Vorrichtung zur Erzeugung von Biogas aus Biomasse, insbesondere aus landwirtschaftlichen Reststoffen, wie Gülle und Fäkalien, im Durchgangsverfahren, mit einer Zuführleitung für frische Biomasse, die in den unteren Bereich eines Gärbehälters mündet, der eine Heizvorrichtung umfasst. Diese Vorrichtung zeichnet sich dadurch aus, dass die Zuführungsleitung aus wärmeleitendem Material besteht, sich durch die vergärende bzw. vergorene Biomasse in den unteren Behälterraum erstreckt und sich von dort in mindestens zwei Rohrleitungen verzweigt, die in einen Ringbereich des Gärbehälters münden und wobei Heizeinrichtungen zu beiden Seiten auf einem Teil dieses Ringbereichs, die frisch eingeführte Biomasse von zwei Seiten aufwärmend, vorgesehen sind. Nachteilig an dieser Vorrichtung ist, dass durch die unkontrollierte Aufheizung der neu eingebrachten Biomasse keine kontrollierte Temperatursteuerung möglich ist. Eine homogene Durchmischung des Fermentationsgutes kann ebenfalls nicht erreicht werden, weil einerseits durch die eingezogenen Zwischenwände und die sich verzweigenden Rohrleitungen des Zuführungsrohres von unten her immer wieder eine unregelmäßige Aufwirbelung des Fermentationsgutes erfolgt und andererseits eine in der Regel nicht einzusetzende Rührvorrichtung in der mit Rohren durchsetzen Apparatur auch keine homogene Durchmischung bewirken kann.

Die Offenlegungsschrift DE 28 21 790 A1 beschreibt einen Gärbehälter mit Vorrichtungen zur kontinuierlichen oder intermittierenden Ausfaulung organischer Substanzen mit Gewinnung von Methan und hygienisch einwandfreiem, geruchsarmen und stickstoffreichen Naturdünger, wobei der Behälter als stehender, oben offener

Zylinder ausgebildet und mit einer Gasglocke versehen ist, oder als oben geschlossener Zylinder bzw. als Kugel mit verschraubtem Mannlochdeckel aus geeignetem Material, vorzugsweise aus Kunststoff gefertigt ist, in dem Rohrleitungen zur Beschickung, Entleerung, Umwälzung des Behälterinhalts mittels eines Pumpstrahls und zur Gasableitung installiert sind. Allerdings ist bei dieser Vorrichtung keine Temperatursteuerungseinrichtung zur flächigen Beheizung vorgesehen. Zudem sind in den abnehmbaren Mannlochdeckel gleichzeitig ein Rührwerk und eine Gasableitung installiert. Deshalb wird eine homogene Durchmischung fortwährend durch aus dem Gärbehälter ausströmendes und um das Rührwerk herumströmendes Biogas behindert.

Ziel der vorliegenden Erfindung ist es, die Nachteile des Standes der Technik zu überwinden und eine Fermentationsvorrichtung bereitzustellen, mit der die Messung von verschiedenen Verfahrensparametern, wie Gärverlauf, Biogasertragspotential, Verweilzeiten und Faulraumbelastungen in ein und der selben Vorrichtung in Reihenuntersuchungen möglich ist. Diese Vorrichtung soll einfach und preisgünstig herzustellen und ohne großen Aufwand an verschiedenen Orten aufstellbar sein. Zudem wird angestrebt, sie so zu gestalten, dass sie sich mühelos öffnen und reinigen lässt und mechanischen Belastungen widersteht.

Hauptmerkmale der Erfindung sind im kennzeichnenden Teil der Ansprüche 1 und 18 angegeben. Ausgestaltungen sind Gegenstand der Ansprüche 2 bis 17.

Die Probleme des Standes der Technik löst eine Fermentationsvorrichtung, die aus einem Behälter, in den Fermentationsmasse und Biomasse über mindestens eine Öffnung einbringbar ist, ferner aus einer Rühreinrichtung zum Rühren von Fermentationsmasse sowie Biomasse und schließlich aus einem Deckel besteht. Erfindungsgemäß sind der Behälter und der Deckel aus einem Kunststoff geformt und im Deckel ist ausschließlich eine Rühreinrichtung angebracht und zwar mittig.

Diese Vorrichtungsmerkmale bewirken, dass der Fermentationsvorgang über die Rühreinrichtung beeinflusst werden kann, wobei deren zentrische Positionierung im Deckel ein Entstehen von Inhomogenitäten im Fermentationsgemisch verhindert. Diese werden auch deshalb vermieden, weil der Deckel nur die Rühreinrichtung enthält. Bei der Fermentation entstehendes Biogas kann durch den Deckel nicht austreten und die Durchmischung des Fermentationsgemisches deshalb nicht behindern. Weil darüber hinaus die zu großen Teilen aus Kunststoff gefertigte Vorrichtung leicht und unzerbrechlich ist, lässt sie sich bequem zu verschiedenen Messorten transportieren. Sie eignet sich deshalb ideal als Modellbioreaktor an dem sich sämtliche Verfahrensparameter, wie Faulraumbelastung, Verweilzeit von Biomasse im Reaktor und Menge an hergestelltem Biogas zusammen und repetitiv unter absolut praxisnahen Bedingungen ermittelten lassen.

Darüber hinaus können viele einzelne erfindungsgemäße Vorrichtungen kostengünstig produziert und nebeneinander beschickt werden. Ergebnisse verschiedener Beschickungsmengen zur Ermittlung einer optimalen Faulraumbelastung sind im Rahmen solcher Reihenuntersuchungen kostengünstig und in kurzer Zeit zu erhalten. Mithin erlaubt die erfindungsgemäße Vorrichtung einen hohen Probendurchsatz

An den Behälterseiten der Fermentationsvorrichtung sind Tragevorrichtungen für den Transport durch mindestens eine Person vorgesehen. Der große Vorteil einer mit diesem Merkmal versehenen erfindungsgemäßen Fermentationsvorrichtung besteht darin, dass sie transportabel ist. Man kann sie ohne weiteres an einem Groß-Fermenter mit Fermentationsgülle als Reaktionsmedium befüllen und anschließend ins Labor zurückbringen um sie in einem Wasserbad zu temperieren oder anders weiter zu behandeln. Ein Umfüllen von Fermentationsgülle aus dem Vorrats-Fermenter in ein Transportgefäß und von diesem Transportgefäß in einen Versuchsfermenter erübrigt sich. Ein Aktivitätsverlust der Fermentergülle ist deshalb auch nicht zu befürchten. Mithin ist durch einfaches Zugeben von Biomasse eine Fermentation gleich im Transportgefäß der Fermentergülle, sprich in der Fermentationsvorrichtung möglich

Erfindungsgemäße Tragevorrichtungen stellen Tragegriffe oder Knaufe dar, die ebenfalls aus einem Kunststoff geformt sein können. Als Kunststoff wird sowohl für den Behälter als auch für die Griffe wenigstens ein Polymer eingesetzt. Meist handelt es sich um ein Polyolefin, wobei insbesondere Polyethylen oder Polypropylen bevorzugt wird. Durch diese Maßnahme ist die erfindungsgemäße Fermentationsvorrichtung deutlich preisgünstiger herzustellen. Während ein Fermentationsbehälter nach dem Stand der Technik ungefähr 1.500 bis 3.000 Euro kostet, insbesondere, weil er aus Glas gefertigt ist, lässt sich der erfindungsgemäße Behälter bereits mit einem finanziellen Aufwand zwischen 100 bis 300 Euro herstellen. Ferner sind erfinderische Behältnisse aus Kunststoff auch deutlich leichter und selbst in gefüllter Form von einer Person noch zu transportieren.

Die Fermentationsvorrichtung ist außerdem mit einer Gastülle ausgestattet. Sie ist für die Gasentnahme vorgesehen und steht mit einem Ausdehnungsgefäß in Verbindung. Druckschwankungen, insbesondere Druckanstiege infolge Biogasproduktion, die für die Fermentations- Bakterienflora teilweise abträglich wären, können so vermieden werden.

Zum Einfüllen oder Entnehmen von Fermentationsmasse weist die Fermentationsvorrichtung ein vorzugsweise gasdicht verschließbares Befüllrohr mit einer Öffnung auf, welches sich in einer Ausgestaltung bis weitgehend zu einem Boden des Behälters erstreckt. Hierdurch ist eine Befüllung des Fermentationsgefäßes unter gleich bleibenden Druckbedingungen möglich.

Die Rühreinrichtung ist in einer Weiterführung mit dem Deckel lösbar verbunden. Sie ist aus einem Rührmotor, einem Getriebe mit einer Welle und einem Rührwerk zusammengesetzt. Dank dieser Ausgestaltung lässt sich nicht nur die mit dem Deckel verbundene Rühreinrichtung als Ganzes vom Behälter abnehmen. Vielmehr ist es auch möglich, die Rühreinrichtung vom Deckel zu trennen und in ihre Komponenten Rührmotor, Getriebe mit Welle und Rührwerk zu zerlegen. Je nach Bedarf lässt sich deshalb die mit dem Deckel verbundene Rühreinrichtung schnell gegen einen ordinären Deckel austauschen oder aber für eine bessere Reinigung vollständig demontieren.

In einer Weiterführung ist die Eintauchtiefe des Rührwerks in den Behälter regulierbar. Bei Bedarf kann es zusätzlich über eine Steuereinheit in der Rühreinrichtung in seiner Drehgeschwindigkeit und alternativ oder zusätzlich in seinem Drehsinn eingestellt werden. Diese Ausgestaltung erhöht die Einsatzmöglichkeiten der Fermentationsvorrichtung beträchtlich, denn je nach Drehgeschwindigkeit und Eindringtiefe lassen sich Biomassen aus unterschiedlichsten Quellen effizient zu Biogas und stickstoffangereichertem Rückstand reagieren.

Das Rührwerk ist bevorzugt u-förmig ausgebildet und weist der besseren Durchmischung wegen zum Deckel gerichtete Arme auf. Diese sind in einer Erweiterung y-formig aufgespreizt.

In einer weitergeleiteten Variante verfügt die erfinderische Vorrichtung über wenigstens eine zusätzliche Befüll- bzw. Entnahmemöglichkeit für Kofermentat, Gärsubstrat oder Biogas. Dies ist besonders dann interessant, wenn Kofermentate also Biomassen aus unterschiedlichsten Quellen verwendet werden oder neben Gülle anteilig auch Klärschlamm als Gärsubstrat eingesetzt wird oder aber Reihenuntersuchungen im kontinuierlichen Betrieb angestrebt werden.

In einer besonders robusten Bauform ist die erfindungsgemäße Einrichtung mit Stabilisierungsstäben versehen. Diese halten den Deckel ortsfixiert und bewirken, dass er sich bei betätigter Rühreinrichtung nicht löst. Ferner wird durch diese Stäbe erreicht, dass durch das Rühren erzeugte Impulse sich nicht ausschließlich auf den Fermentationsbehälter übertragen. Auf diese Weise verhindert man, dass sich eine Fermentationsvorrichtung bei sich drehendem Rührwerk selbsttätig im Raum bewegt oder infolge starker Auslenkung umfällt.

Abgerundet wird die Fermentationsvorrichtung in einer Weiterleitung, die an ihr Messfühler vorsieht. Diese sind vorzugsweise als Elektroden ausgebildet, so dass auch mit einem Gärprozess einhergehende elektrochemische Vorgänge studiert werden können.

Ein wesentlicher Bestandteil der Erfindung ist ferner ein Verfahren zur Fermentierung von Biomasse insbesondere mit der neu bereitgestellten Vorrichtung. Dieses umfasst ein Befüllen der Vorrichtung mit Fermentationsmasse, ein anschließendes Beschicken mit Biomasse, ein Temperieren der verschlossenen Vorrichtung und ein isobares Ableiten des entstandenen Biogases. Erfindungsgemäß wird die Vorrichtung hierzu nach dem Befüllen mit Fermentationsmasse an ihren Tragevorrichtungen gefasst und zur weiteren Beschickung, Temperierung und Gasableitung an einen anderen Ort verbracht.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:

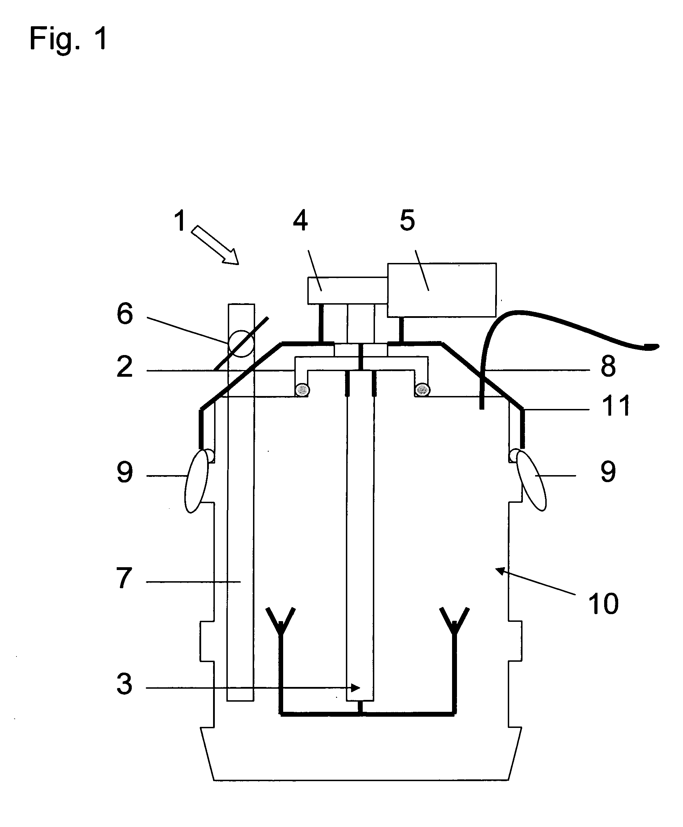

- Fig. 1: eine Frontalansicht einer Fermentationsvorrichtung

Die erfindungsgemäße Fermentationsvorrichtung in Fig. 1 ist mehrstückig aufgebaut. An einen am Behälter 10 vorgesehenen Deckel 2 ist eine Rührvorrichtung zentrisch und in Alleinstellung angebracht. Sie besteht aus einem Rührer 3, einem Getriebe 4 und einem Rührmotor 5. Ein ebenfalls in den Behälter 10 eingelassener und mit einem Befüllrohr 7 versehener Einfüllstutzen mit Kugelhahn 6 dient zur Befüllung mit Fermentationsmasse. Ferner ist eine Tülle 8 zur Gasentnahme vorgesehen. Schließlich sind Tragegriffe 9 für den Transport durch mindestens eine Person am Gärbehälter 10 angebracht.

Somit besteht ein erfindungsgemäßer Fermentationsbehälter 1 aus einem gasdichten Kunststoffbehälter 10 mit einem hermetisch aufschraubbaren, aufclipsbaren oder verrastbaren Deckel 2, in dem ausschließlich die Rühreinrichtung fixiert ist. In einer weiteren Bauform ist sie lösbar im Deckel 2 angebracht und in einer besonders leistungsfähigen Variante ist die Rühreinrichtung im Deckel 2 lösbar aber festgelegt angeordnet. Festgelegt bedeutet, dass sie nicht nur im Deckel 2 verschraubt oder eingeclipst ist, sondern durch eine zusätzlich vom Nutzer zu betätigende Sicherung arretiert und damit gegen Ablösung vom Deckel 2 geschützt ist. Darüber hinaus enthält der Behälter 10 eine gasdichte Befülleinrichtung 7 zur Aufnahme von Biomasse, wie Silage, Weizen- oder Maisschrot, kleingehäckselten Biomaterialien und ähnlichem. Ferner ermöglicht die Befülleinrichtung eine Be- und Entnahme von Flüssigkeit sowie das Abziehen von aus der Flüssigkeit ausgasenden Teilchen mittels einer Spritze.

In die transportable Fermentationsvorrichtung 1, die in etwa die Größe eines Kraftstoffkanisters oder eines Behälters, wie er zur Obstweinherstellung angeboten wird, hat, kann zunächst Fermentationsmasse aus einem großen Behälter eingelassen werden, wobei unter Fermentationsmasse i.d.R. Fermentationsgülle oder Klärschlamm verstanden wird. Man öffnet hierzu den Kugelhahn am Einfüllstutzen 6 und lässt beispielsweise Gülle durch das Befüllrohr 7 auf den Boden der Vorrichtung 1 strömen. Anschließend wird der Fermentationsreaktor an anderem Ort, beispielsweise in einem Labor durch eine mit dem Deckel 2 verschließbare Öffnung mit Biomasse befüllt und gasdicht verschlossen, so dass ein Fermentationsprozess in Gang gesetzt wird. Dieser Prozess wird dadurch unterstützt, dass im Deckel 2 ein Rührwerk fixiert oder lösbar festgelegt ist. Mit der Zeit entsteht nun Biogas, das über die Gastülle 8 in einen Gasaufnahmebehälter strömt.

Unter physiologischen Bedingungen sind metabolisierende Bakterien in der Fermentergülle besonders aktiv. Deshalb stellt man den Reaktor 1 in ein 38°C heißes Wasserbad um die Fermentation zu fördern.

Für die Zugabe oder Entnahme von Fermentationsmasse, insbesondere Fermentationsgülle, lässt sich der Einfüllstutzen 6 auch während der Fermentation regelmäßig öffnen und durch Einführen eines Probennehmers in das Tauchrohr 7 ein Aliquot der im unteren Behälterteil befindlichen Fermentermasse entnehmen. In einer besonderen Ausgestaltung sind der Einfüllstutzen 6 und das an ihn angeschlossene Tauchrohr dabei so ausgeführt, dass sich im Moment der Entnahme oder Zugabe von Fermentergülle die Druckverhältnisse in der Fermentationsvorrichtung 1 nicht ändern, wodurch eine den Innendruck der Vorrichtung 1 unverändert lassende Befüllung des Behälters 10 möglich ist.

Die schlauchförmige Gastülle 8 reicht nur in den oberen Teil des Fermentationsbehälters 10 hinein, um ausschließlich vom während der Fermentation entstehenden Gasgemisch (Methan und Kohlendioxid in etwa gleichen Anteilen) umströmt zu werden. Ein Kontakt mit Biomasse oder Fermentermasse kann so weitestgehend vermieden werden. Die Tülle 8 steht mit einem Ausdehnungsgefäß in Strömungsverbindung. Hierbei handelt es sich um einen Folienbeutel, also um einen elastischen Balg oder Ballon beispielsweise aus Kautschuk, der zur Stabilisierung in einer Ausführung mit Metall bedampft ist. Doppelseitige Bedampfung macht einen solchen Balg besonders widerstandsfähig gegen den Übertritt von Biogas in die Atmosphäre. Gängige Ausdehnungsgefäße sind unter dem Handelsnamen Alubag™ erhältlich und werden als aluminiumbeschichtete Ballons bevorzugt eingesetzt.

Zu Beginn der Fermentation baut sich durch das entstehende Biogas in der Fermentationsvorrichtung ein Gasdruck auf. Das Ausdehnungsgefäß gewinnt mit zunehmender Biogasmenge an Volumen und garantiert so über lange Zeiträume einen nahezu konstant bleibenden Gasdruck im Fermentationsbehälter 1. Dies ist für eine gute Verstoffwechselung der im Reaktor 1 befindlichen Biomasse durch die in der Fermentergülle vorliegenden Methan erzeugenden Bakterien wesentlich.

Man nimmt das Ausdehnungsgefäß nach Befüllung mit entstandenem Biogas, beispielsweise im Tagesabstand von der Gastülle 8 ab und verbindet es beispielsweise mit einem Trommelgaszähler. Dieser bestimmt die Menge des entstandenen Gases. Zur weiteren Analyse leitet man das Gas dann in ein IR-Spektrometer ein. Hier wird ein für jedes Gasgemisch charakteristisches Infrarotspektrum aufgenommen.

In dem im oberen Teil des Fermentationsbehälters 1 befindlichen Deckel 2 ist ein Getriebe 4 vorgesehen. Dieses ist über eine Welle mit einem Rührwerk 3, welches in einer Variante U-förmig ausgestaltet ist, verbunden. Dieses Rührwerk 3 gewährleistet, dass die über den Einfüllstutzen 6 eingefüllte Fermentergülle und die über die durch den Deckel 2 verschließbare Öffnung aufgegebene Biomasse innig miteinander vermischt werden. Den Grad der Vermischung beeinflusst man durch Einschieben des Rührwerks 3 in die Biomasse oder entsprechendes Herausziehen. Je tiefer sich der Rührer 3 in der Biomasse befindet, desto mehr biologisches Material wird umgewälzt und umso höher ist die Gasproduktion. Dies gilt auch wenn der Rührer über ein an der Rührvorrichtung vorgesehenes Steuerelement in seiner Drehgeschwindigkeit bzw. seiner Drehrichtung beeinflusst wird.

Die zum Deckel gerichteten Arme des Rührwerks 3 sind bevorzugt parallel zur Welle oder aber in Richtung der Wände des Behälters 10 ausgerichtet. Die y-förmige Aufspreizung der Arme ist nicht nur in einer Ebene sondern in allen drei Raumrichtungen möglich, so dass ein Arm, wenn man von oben auf die geöffnete Vorrichtung 1 schaut, die Form eines Kreuzes besitzt.

Die am Fermentationsbehälter seitlich vorgesehenen Tragegriffe 9 gestatten es, die Fermentationseinrichtung 1 zu transportieren und mühelos an jedem beliebigen Ort aufzustellen. Diese Tragegriffe 9 sind als Henkel so dimensioniert, dass die Fermentationsbehälter 10 auch bei vollständiger Befüllung mit Biomasse und Fermentationsmasse sowie bei eingeführter Rührvorrichtung angehoben werden kann, ohne dass es zu Abnutzungserscheinungen am Henkel 9 kommt. Ferner besitzen die Tragegriffe in einer Ausgestaltung Fingermulden und in einer noch weiteren Ausbildung der Erfindung sind diese Fingermulden gepolstert ausgeführt, um den Tragekomfort nochmals zu erhöhen.

Da der Behälter 10, die Tragegriffe 9 und meist auch der Deckel 2 aus Kunststoff und mechanische Teile aus Edelmetall oder Stahl gefertigt sind, lässt sich die gesamte Vorrichtung 1 leicht reinigen. Ferner ist sie bruchsicher und leichtgewichtig.

Ist die erfindungsgemäße Vorrichtung nur zu einem geringen Teil mit Biomasse oder Fermentationsmasse gefüllt, liegt die relativ schwerere Rühreinrichtung auf dem recht leichten Fermentationsbehälter 10 auf. Mithin kann es in einem solchen Fall bei hohen Drehgeschwindigkeiten des Rührers 3 dazu kommen, dass der Fermentationsreaktor 1 zu Hüpfbewegungen neigt oder zu schwanken beginnt. Bei gut gefülltem Reaktor 1 kann sich andererseits die Rühreinrichtung im Deckel 2 lockern, insbesondere wenn sehr dichte Biomasse umgesetzt wird.

Um all dem entgegenzuwirken, verfügt die Vorrichtung 1 in einer Weiterführung über Stabilisierungsstäbe 11. Diese sind aus einem starren vorzugsweise schweren Werkstoff gefertigt und stützen sich auf dem Behälter 10 ab. Sie zwängen den Deckel 2 samt Rühreinrichtung zwischen sich ein, so dass er relativ zum Behälter 10 nicht beweglich ist. In einer Ausführungsform sind sie fest mit dem Behälter 10 verbunden und pressen sich gegen den Deckel 2. In einer anderen Bauform sind sie am Deckel 2 befestigt und liegen stramm auf dem Behälter 10 auf. In einer dritten Bauvariante schließlich sind sie sowohl am Deckel 2 als auch am Behälter 10 befestigbar oder festlegbar.

Die auch als Grob-Fermentat oder Kofermentat bezeichnete Biomasse umfasst Silage, Abfälle, frische Pflanzen, Kompost, kleingehäckselten Mais sowie sämtliche weitere organische oder pflanzliche Reste, die dem Anwender zur Verfügung stehen (auch Pflanzenmaterial aus Sukkulenten, wie Kakteen). Ferner lassen sich einem Fermentationsprozess auch Presskuchen bereits fermentierter Biomasse zusetzen um die Parameter Gärzeit oder Faulraumbelastung zu verändern. Weil fermentierte Biomasse weniger stark zur Metabolisierung durch den Gärprozess neigt, entsteht weniger dem Fermentierprozeß abträgliche Essigsäure und somit können größere Mengen an Biomasse in den Reaktor 1 verbracht, also eine größere Faulraumbelastung gewählt werden, ohne dass es zum Stillstand des Fermentationsprozesses kommt

Die erfindungsgemäße Fermentationsvorrichtung 1 ist im Wesentlichen für den Einsatz in Forschungseinrichtungen gedacht, da man über die Gastülle 8 die Gasproduktion beobachten, über den Einfüllstützen 6 Fermentergülle be- und entnehmen und über den mit der Rühreinrichtung versehenen Deckel 2 Biomasse einfüllen und stark oder schwach durchmischen kann. Es ist also möglich, alle bei einer Fermentation sich ändernden Parameter an der Vorrichtung 1 zu studieren.

Ferner lässt diese sich für eine unbewegte Fermentation platzsparend abstellen, denn der erfindungsgemäße mit der Rührvorrichtung verbundene und daher voluminöse Deckel 2 lässt sich mühelos durch eine simple Kappe, sprich einen Transportdeckel ersetzten. Schließlich lässt sich der Fermentationsapparat 1 leicht an verschiedene Orte verbringen, weil er von einer Person getragen werden kann. Er umfasst ein Volumen von 10 bis 30 Liter, wobei ein Reaktionsraum von 20 Litern bevorzugt ist.

Durch dieses geringe Volumen wird eine gute Durchmischung und somit eine ausgeprägte Gasbildung und Gasbewegung im Reaktionsgefäß ermöglicht.

In einer ergänzten Bauform der Vorrichtung sind weitere gasdichte Verschraubungen am Behälter 10 vorgesehen. Diese nehmen Messsonden verschiedener Art, (z.B. pH-Elektroden oder Doppelplatin Elektroden zur Redox Messung), Heizvorrichtungen, weitere Rühraggregate sowie verschiedene Zugabe- und Entnahmevorrichtungen für Kofermentat oder Klärsubstrat gasdicht auf, wodurch sich die Einsatzmöglichkeiten der erfindungsgemäßen Vorrichtung 1 als Forschungsreaktor nochmals vergrößern. Im besondern ist es mit dieser Erweiterung möglich, unterschiedlichste Mengen und Arten vergärbaren Materials zu verschiedensten Zeiten der Vorrichtung zuzugeben oder aus ihr zu entnehmen. Für fast alle Materialien lassen sich mit dieser Bauform sogar Reihenuntersuchungen zu Faulraumbelastungen und Verweilzeiten preisgünstig durchführen

Die Erfindung ist nicht auf eine der vorbeschriebenen Ausführungsformen beschränkt, sondern in vielfältiger Weise abwandelbar.

So lassen sich eigenbewegte Teile wie Getriebe 4, Rührmotor 5 und Rührwerk 6 nicht nur aus Stahl bzw. anderen Metallen sondern auch aus Kunststoff ausführen. Ferner haben sich neben Polyolefinen auch andere Kunststoffe wie Polycarbonat, Polyvinylchlorid oder Polyurethan als gute Behältermaterialien erwiesen.

### Ausführungsbeispiel 1

Die erfindungsgemäßen Behälter 10 werden mit einer definierten Menge (ca. 15 kg) Fermentergülle bekannter Zusammensetzung am Großreaktor befüllt und mit dem Deckel 2 oder einer Kappe verschlossen. Im Labor ersetzt man die gegebenenfalls aufgesetzte Kappe durch den Deckel 2 mit Rührwerk und temperiert den Behälter 10 im Wasserbad. Über den Einfüllstutzen 6 führt man dem Reaktor 1 eine vorab berechnete oTS-Menge [oTS steht für kg organische Trockensubstanz/m³ Fermentervolumen (bzw. g oTS/I)] an Kofermentat nach zeitlicher Vorgabe in mehreren festgelegten Portionen zu. Am nächsten Tag wird je nach Verweilzeit eine bestimmte Menge fermentierter Substanz abgesaugt, diese durch eine auf OTS bezogene Kofermentatmenge ersetzt und das durch Probenabsaugung reduzierte Fermentationsvolumen in der Vorrichtung 1, wenn nötig, durch Wasserzugabe korrigiert. Entstandenes Biogas wird in täglich zu wechselnden Aluminiumgasbags aufgefangen und mittels Trommelgaszähler und IR-Spektrometer quantitativ und qualitativ erfasst.

### Ausführungsbeispiel 2

Bei den Bestimmungen von Gärverlauf und Biogasertragspotential im Batch-Betrieb, d.h. beim gleichzeitigen Einsatz mehrerer Vorrichtungen 1 wird der Einfüllstutzen 6 nicht benutzt. Die einmalige Kofermentatzugabe erfolgt hier bei der Befüllung durch die von Deckel 2 verschließbare Öffnung. Aus dem Gärverlauf (in der Regel Tagesmessung) ergibt sich die Gärgeschwindigkeit (Umsatzgeschwindigkeit), die ein Schlüsselparameter zur Festlegung des zu testenden Verweilzeitbereiches darstellt. Standards ermöglichen die Beurteilung der Gülleaktivität. Mit der neuen erfindungsgemäßen Vorrichtung 1 ist es möglich, die oben genannten Parameter in Reihenuntersuchungen schnell, mit einfachen Mitteln und kostengünstig zu erfassen.

Die erfindungsgemäßen Fermentationsvorrichtungen 1 werden nach dem Befüllen mit Fermentermasse, insbesondere Fermentergülle und Biomasse in Temperaturbäder gestellt, welche beliebig groß sein können. Von den Bädern sind einige für jeweils 10 der 20 Liter Gärbehälter geeignet, andere fassen über 20 Behälter. Allerdings müssen in allen einzelnen Bädern ein Reaktor mit Impfschlamm ohne Zusatz und ein Reaktor mit Impfschlamm und Standard vorhanden sein. Mit Hilfe des Standards wird ein Aktivitätskoeffizient ermittelt und damit der Vergleich mit anderen Gärversuchen möglich.

Jedes einzelne der isolierten Temperierbäder wird indirekt beheizt. Heizschläuche an den Wandungen sind mit der Hausheizung verbunden und werden über Thermostate mit einer Genauigkeit von +/- 1 °C geregelt. Das Heizungswasser (70°C) erlaubt je nach Bedarf einen Betrieb im mesophilen (30 bis 45°C) oder im thermophilen (> 50°C) Bereich. Wenn eine Fermentation mesophil erfolgen soll (wie bei den meisten Proben), werden alle Bäder auf eine Temperatur von 38°C +/- 1°C eingestellt.

Man erkennt, dass die Erfindung sich auf eine Fermentationsvorrichtung 1 bezieht. Diese umfasst einen Behälter 10, in den Fermentationsmasse und Biomasse über mindestens eine Öffnung einbringbar ist. Sie weist ferner eine Rühreinrichtung zum Rühren von Fermentationsmasse und Biomasse sowie einen Deckel 2 auf. In diesen Deckel 2 ist ausschließlich die Rühreinrichtung und zwar in dessen Mitte eingebracht. Ferner sind der Behälter 10 und der Deckel 2 aus einem Kunststoff geformt.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

### Bezugszeichenliste

- 1: Fermentationsvorrichtung
- 2: Deckel
- 3: Rührer, Rührwerk
- 4: Getriebe
- 5: Rührmotor
- 6: Einfüllstutzen mit Kugelhahn
- 7: Befüllrohr, Tauchrohr
- 8: Tülle, Gastülle
- 9: Tragegriffe, Tragevorrichtungen
- 10: Behälter, Fermentationsbehälter
- 11: Stabilisierungsstäbe

## Patentansprüche

1. Fermentationsvorrichtung umfassend einen Behälter (10), in den Fermentationsmasse und Biomasse über mindestens eine Öffnung einbringbar ist, eine Rühreinrichtung zum Rühren von Fermentationsmasse und Biomasse sowie einen Deckel (2), **dadurch gekennzeichnet, dass** der Behälter (10) und der Deckel (2) aus einem Kunststoff geformt sind und dass im Deckel (2) ausschließlich eine Rühreinrichtung und zwar mittig angebracht ist.

2. Fermentationsvorrichtung nach Anspruch 1, **dadurch gekennzeichnet, dass** an den Behälterseiten Tragevorrichtungen (9) für den Transport durch mindestens eine Person vorgesehen sind.

3. Fermentationsvorrichtung nach Anspruch 1 oder 2, **dadurch gekennzeichnet, dass** der Kunststoff ein Polyolefin, insbesondere ein Polyethylen oder Polypropylen ist.

4. Fermentationsvorrichtung nach einem der Ansprüche 1 bis 3, **dadurch gekennzeichnet, dass** sie mit einer Gastülle (8) ausgestattet ist.

5. Fermentationsvorrichtung nach Anspruch 4, **dadurch gekennzeichnet, dass** die Gastülle (8) mit einem Ausdehnungsgefäß in Verbindung steht.

6. Fermentationsvorrichtung nach einem der Ansprüche 1 bis 5, **dadurch gekennzeichnet, dass** die Vorrichtung (1) ein Befüllrohr (7) mit einer Öffnung aufweist.

7. Fermentationsvorrichtung nach Anspruch 6, **dadurch gekennzeichnet, dass** das Befüllrohr (7) gasdicht verschließbar ist.

8. Fermentationsvorrichtung nach Anspruch 6 oder 7, **dadurch gekennzeichnet, dass** das Befüllrohr (7) sich bis weitgehend zum Boden des Behälters erstreckt.

9. Fermentationsrohr nach einem der Ansprüche 1 bis 8, **dadurch gekennzeichnet, dass** die Rühreinrichtung mit dem Deckel (2) lösbar verbunden ist.

10. Fermentationsvorrichtung nach einem der Ansprüche 1 bis 9, **dadurch gekennzeichnet, dass** die Rühreinrichtung aus einem Rührmotor (5), einem Getriebe (4) mit einer Welle und einem Rührwerk (3) zusammengesetzt ist.

11. Fermentationsvorrichtung nach Anspruch 10, **dadurch gekennzeichnet, dass** die Eintauchtiefe des Rührwerks (3) in den Behälter (10) einstellbar ist.

12. Fermentationsvorrichtung nach Anspruch 10 oder 11, **dadurch gekennzeichnet, dass** das Rührwerk (3) über eine Steuereinheit in der Rühreinrichtung in seiner Drehgeschwindigkeit und alternativ oder zusätzlich in seinem Drehsinn einstellbar ist.

13. Fermentationsvorrichtung nach einem der Ansprüche 10 bis 12, **dadurch gekennzeichnet, dass** das Rührwerk (3) u-förmig ausgebildet ist.

14. Fermentationsvorrichtung nach einem der Ansprüche 10 bis 13, **dadurch gekennzeichnet, dass** das Rührwerk zum Deckel (2) gerichtete Arme aufweist.

15. Fermentationsvorrichtung nach Anspruch 14, **dadurch gekennzeichnet, dass** die Arme zum Deckel hin y-förmig aufgespreizt sind.

16. Fermentationsvorrichtung nach einem der Ansprüche 1 bis 15, **dadurch gekennzeichnet, dass** sie wenigstens eine zusätzliche Befüll- bzw. Entnahmemöglichkeit für Kofermentat, Gärsubstrat sowie Biogas aufweist.

17. Fermentationsvorrichtung nach einem der Ansprüche 1 bis 16, **dadurch gekennzeichnet, dass** sie mit Stabilisierungsstäben (11) versehen ist.

18. Fermentationsvorrichtung nach einem der Ansprüche 1 bis 17, **dadurch gekennzeichnet, dass** sie Messfühler enthält, die bevorzugt als Elektroden ausgebildet sind.

19. Verfahren zur Fermentierung von Biomasse insbesondere mit einer Vorrichtung (1) nach einem der Ansprüche 1 bis 18,
- bei dem Fermentationsmasse in die Vorrichtung (1) gefüllt wird,
- diese mit Biomasse beschickt wird,
- die verschlossene Vorrichtung (1) temperiert wird
- und entstandenes Biogas isobar abgeleitet wird,
**dadurch gekennzeichnet, dass** die Vorrichtung (1) nach dem Befüllen mit Fermentationsmasse an ihren Tragevorrichtungen (9) gefasst und zur weiteren Beschickung, Temperierung und Gasableitung an einen anderen Ort verbracht wird.
